# EUROPEAN PATENT SPECIFICATION

(11) **EP 1 079 826 B1**
(45) Date of publication and mention of the grant of the patent: **05.03.2003**
(21) Application number: 99926420.3
(22) Date of filing: 26.05.1999
(51) Int. Cl.: A61K 31/40, C07D 209/34, A61P 9/00, A61P 17/06, A61P 25/28, A61P 31/00, A61P 31/12

(54) **USE OF INDIGOID BISINDOLE DERIVATIVES FOR THE MANUFACTURE OF A MEDICAMENT TO INHIBIT CYCLIN DEPENDENT KINASES**
VERWENDUNG VON INDIGOÄHNLICHEN BISINDOLEN ZUR HERSTELLUNG EINES ARZNEIMITTELS ZUR HEMMUNG CYCLIN ABHÄNGIGER KINASEN
UTILISATION DE DERIVES BISINDOLIQUES D'INDIGOIDES POUR LA PREPARATION D'UN MEDICAMENT SERVANT A INHIBER DES KINASES DEPENDANTES DE CYCLINE

(30) Priority: 29.05.1998 EP 98109845; 19.03.1999 EP 99105693
(43) Date of publication of application: 07.03.2001
(73) Proprietor: CNRS, Centre National de la Recherche Scientifique, 75005 Paris (FR); Eisenbrand, Gerhard, Prof. Dr., 69120 Heidelberg (DE)
(72) Inventor: EISENBRAND, Gerhard, D-69120 Heidelberg (DE); MARKO, Doris, D-67657 Kaiserslautern (DE); TANG, Weici, D-67663 Kaiserslautern (DE); MEIJER, Laurent, F-29680 Roscoff (FR); HöSSL, Ralph, D-67659 Kaiserslautern (DE)
(74) Representative: Müller-Boré & Partner Patentanwälte
(86) International application number: EP9903625
(87) International publication number: WO99062503

(56) References cited:
- WO-A-98/07695
- HOESSEL, RALPH ET AL: "Indirubin, the active constituent of a Chinese antileukemia medicine, inhibits cyclin-dependent kinases" NAT. CELL BIOL. (1999), 1(1), 60-67 , XP002122993
- DATABASE CHEMABS [Online] CHEMICAL ABSTRACTS SERVICE, COLUMBUS, OHIO, US JI, XIUJUAN ET AL: "Antineoplastic effect of indirubin derivatives and their structure-activity relations" retrieved from STN Database accession no. 103:98313 XP002123718 & YAOXUE XUEBAO (1985), 20(2), 137-9 ,
- DATABASE CHEMABS [Online] CHEMICAL ABSTRACTS SERVICE, COLUMBUS, OHIO, US ANON.: "Phase II clinical trial on meisoindigo in the treatment of chronic myelogenous leukemia" retrieved from STN Database accession no. 127:214760 XP002123719 & ZHONGHUA XUEYEXUE ZAZHI (1997), 18(2), 69-72 ,
- DATABASE CHEMABS [Online] CHEMICAL ABSTRACTS SERVICE, COLUMBUS, OHIO, US GU, Y. C. ET AL: "Synthesis of some halogenated indirubin derivatives" retrieved from STN Database accession no. 112:178548 XP002123720 & YAOXUE XUEBAO (1989), 24(8), 629-32 ,
- PATENT ABSTRACTS OF JAPAN vol. 010, no. 148 (C-350), 29 May 1986 (1986-05-29) & JP 61 007254 A (ISUKURA SANGYO KK), 13 January 1986 (1986-01-13)
- HUNTER T ET AL: "CYCLINS AND CANCER II: CYCLIN D AND CDK INHIBITORS COME OF AGE" CELL,US,CELL PRESS, CAMBRIDGE, NA, vol. 79, page 573-582 XP002069793 ISSN: 0092-8674
- ARTUR BURGER ET AL.: "Pharmaceutisches Wörtebuch" 1998 , DE GRUITER , BERLIN XP002123717 page 704-705

## Description

The present invention relates to the use of indigoid bisindole derivatives for the manufacture of a medicament for inhibiting cyclin dependent kinases for the treatment of psoriasis, cardiovascular diseases, infectious diseases, nephrology, neurodegenerative disorders and viral infections, all of which are connected to the loss of cell proliferation control, particularly CDK1 CDK2, CDK4 and CDK5, more particularly ATP:Proteinphosphotransferase p 34^{cdc2} (CDK1).

Indigoid bisindoles comprise a spectrum of natural dye stuffs. Many of these can be obtained from plants. Accordingly, indirubine, indigo and isoindigo are natural products which can be obtained from different plants: namely, Baphicacanthus cusia (Acanthaceae), Indigofera suffruticosa (Fabaceae), Isatis indigotica (Brassicaceae) and others. Indican, a glycoside which is found in plants, gives glucose and 3-hydroxyindole due to acidic or enzymatic hydrolysis. 3-Hydroxyindole is converted by air-oxidation into indigo and its isomers. Indigo naturalis (Chinese: quingdai) is the natural blue dye obtained from plant material, e.g. Isatis indigotica (Brassicaceae). Indirubine, an isomer of indigo, can be found in Indigo naturalis in an amount of up to 60% (Falbe J. & Regitz M., Römpp Chemie Lexikon (1992), 9. Aufl., Stuttgart, Georg Thieme Verlag). It occurs also in Isatis tinctoria in an amount of up to 5% which is indigenous to Central Europe (Gelius R., Z. Chem., 20, (1980), 340-341).

Indigo naturalis is reported to be used in traditional Chinese medicine as a hemostatic, antipyretic, anti-inflammatory and sedative agent in the treatment of bacterial and viral infections. Antileukemic effects of Indigo naturalis have also been reported, with indirubine being the effective principle (Ji X. et al., Acta Pharm. Sin., 16, (1981), 146-148; Gan W. J. et al., J. Hematol., 6, (1985), 611-613). Furthermore, derivatives of indirubine are known for a long time as dyes of low persistence.

Few, structurally quite different types of p34^{cdc2} inhibitors have been described up to now. They are either of natural origin or derived from natural compounds and show varying degrees of inhibitory activities. Examples are Staurosporine, an alkaloid from Streptomyces sp, Butyrolactone-I from Aspergillus terreus var., Flavopiridol, a novel promising anti-tumour agent derived by partial synthesis from a parent structure found in the indian plant Dysoxylum binectariferum, 9-Hydroxyellipticin from the plants Ochrosia elliptica and Ochrosia acuminata, the purine derivatives olomoucine, roscovitine and isopentenyladenine and some peptides. The mechanism of these compounds is based on competitive inhibition of ATP binding (Meijer L., Trends in Cell Biology, 6, (1996), 393-397).

For determination of the IC 50-values: p34^{cdC2}/cyclin B was purified from M phase starfish (Marthasterias glacialis) oocytes by affinity chromatogaphy on p9^{CKShs1}-Sepharose beads, from which it was eluted by free p9^{cKShs1} as described (Meijer et al., Eur. J. Biochem., 243, (1997), 527-536). It was assayed with 1 mg histone H1 (Sigma type III-S)/ml, in the presence of 15 µM [γ-³²P] ATP (3,000 Ci/mmol; 1mCi/ml) in a final volume of 30µl. After 10 min. incubation at 30°C, 25 µl aliquots of supernatant were spotted onto 2.5 x 3 cm pieces of Whatman P81 phosphocellulose paper, and after 20 sec., the filters were washed five times (for at least 5 min. each time) in a solution of 10 ml phosphoric acid/liter of water. The wet filters were transferred into 6 ml plastic scintillation vials, 5 ml ACS (Amersham) scintillation fluid was added and the radioactivity measured in a Packard scintillation counter. The kinase activity was expressed in pmoles phosphate incorporated into histone H1/10 min incubation or in % of maximal activity.

The strongest inhibitors of p34^{cdc2} are derivatives of staurosporine (IC₅₀ = 0,003-0,03 µM). The selectivity of these inhibitors is, however, rather poor. They also show more or less potent inhibitory activity to quite a wide range of cellular kinases. Table 1 shows the specifity of known chemical inhibitors of cyclin-dependent kinases (IC₅₀-values given in µM) (cf. Meijer L., Trends in Cell Biology, 6, (1996), 393-397).

**Tab. 1:**

| Enzyme | Staurosporine | UCN-01 | Butyro-lactone-I | Flavopiridol | Olomoucin | Roscovitine | 9-Hydroxy-ellipticine |
|---|---|---|---|---|---|---|---|
| CDK1 | 0.003- | 0.031 | 0.60 | 0.40 | 7 | 0.65^{a} | ca. 1 |
| | 0.009 | | | | | | |
| CDK2 | 0.007 | 0.030 | 1.50 | 0.40 | 7 | 0.70 | ND |
| CDK4 | < | 0.032 | no | 0.40 | >1000 | >100 | ND |
| | 10.000 | | effect | | | | |
| MAPK | 0.020 | 0.910 | 94 | ND | 30 | 30 | ND |
| PKA | 0.008 | ND | 260 | 145 | > 2000 | >1000 | ND |
| PKG | 0.009 | ND | ND | 6 | > 2000 | >1000 | ND |
| PKC | 0.005 | 0.007 | 160 | ND | >1000 | >100 | ND |
| Tyrosine- | 0.006 | ND | >590 | 25 | 440 | 70 | ND |
| kinase | (EGF-R) 0.006 (Src) | | (EGF-R) | (EGF-R) | (EGF-R) | (I-R) | |

| | | | | | | | |
|---|---|---|---|---|---|---|---|
| CDK: cyclin-dependent kinase; EGF-R: epidermal growth factor receptor tyrosine kinase; I-R: insulin receptor-tyrosine kinase; MAPK: mitogene-activated protein kinase; ND: not determined; PKA: cAMP-dependent protein kinase; PKG: cGMP-dependent protein kinase. ^{a}IC₅₀-value for racemic mixture; IC₅₀ for (R)-roscovitin is 0.45 µM. | | | | | | | |

Cyclins and cyclin dependent kinases (CDK) have an essential role for driving the cell through the cell cycle (cell division cycle, cdc). During the cell division cycle, oscillations in concentrations and activities of cyclins are observed. This applies, e.g. to cyclins D and E in the so-called G1-phase of the cell cycle and to cyclinA (S- and M-Phase) and cyclinB (G2- and M-Phase).

The cyclin dependent kinases are activated by association with a member of the cyclin-family. Up to now, eight human CDKs have been described: CDK1 ( = p34^{cdc2}), CDK2 to CDK8. CDK-proteins consist of a catalytic subunit and a regulatory subunit, the cyclins (Meijer et al., Eur. J. Biochem., 243, (1997), 527-536). Every step of the cell division cycle is regulated by specific CDK/cyclin complexes which ascertain a strict control. Important checkpoints are at the transition from G1 phase to S phase and from G2 phase to M phase (Pines J., Cancer Biology, 6, (1995), 63-72). The p34^{cdc2}/cyclinB complexes are important components at the G₂-M-checkpoint.

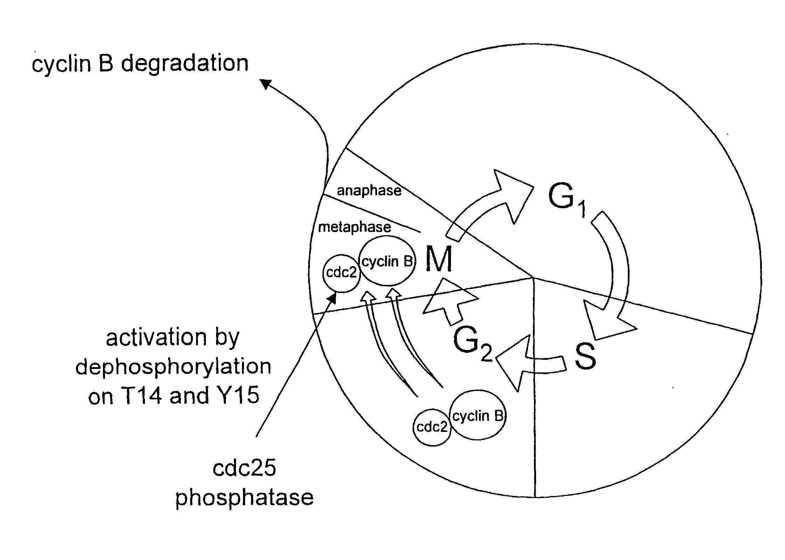

Fig. 1 shows the points of action of the cyclin CDK complex cdc2/cyclinB in the cell divsion cycle (M = mitosis, cell division; G = gap; S = synthesis; interphase = G₁ + S + G₂).

The cdc2/cyclinB complexes are the primary active protein kinases in mitosis. They accumulate in an inactive state in the cytoplasm in interphase cells, and are then rapidly activated by cdc25 phosphatase and translocated into the nucleus at the beginning of mitosis (Pines J., Hunter J., Cell Biol., 115(1), (1991),1-17). CyclinB is degraded at the metaphase-anaphase transition, inactivating cdc2, which is necessary for exit from mitosis (Glotzer et al., Nature, 349(6305), (1991),132-138; Murray A.W., Nature, 339(6222), (1989); 280-286; Surana U. et al., Cell, 65(1), (1991), 145-161). Multiple changes of CDK proteins and their regulators are associated with the development of human tumours (Cordon-Cardo C., Am. J. Pathol., 147(3), (1995), 545-560).

STN Database No. 103:98313 XP002123718 and Yaxue Xuebao (1985), 20(2), 137-9) describes a series of N-mono- or N,N'-disubstituted indirubine, indigo and isoindigo derivatives which showed a higher anti tumor activity (Walker 256 carcinoma in rats) than indirubine itself. STN Database No. 127:214760 XP-002123719 and Zhonghua Xueyexue Zashi (1997), 18(2), 69-72) describes a study in which cases of chronic myelogenous leukemia (CLM) were treated with meisoindigo that is N-Methyl-isoindigo and as such an isoindigo derivative. The total response rate was 90.1% and the complete and partial remission rate was 81.3%. STN Database No. 112:178548 XP-002123720 and Yaoxue Xuebao-(1989), 24(8), 629-32) relates to 5-iodo-, 5-bromo-, 5-chloro- and 5,7-dibromo-indirubine which have been prepared by condensation of haloisatins with 3 hydroxyindole. 5-Iodo-indirubine showed antitumor activity superior to that of indirubine.

Thus, the technical problem underlying the present invention is to provide new inhibitors for cyclin dependent kinases, particularly CDK 1, CDK2, CDK 4 and CDK 5, more particularly ATP:Proteinphosphotransferase p34^{cdc2} (CDK1), which exhibit a high selectivity as well as high efficiency compared to the inhibitors known in the art.

The solution to the above technical problem is achieved by the embodiments
characterized in the claims.

The present invention relates to the use of indigoid bisindole derivatives for the manufacture of a medicament for inhibiting cyclin dependent kinases for the treatment of psoriasis, cardiovascular diseases, infectious diseases, nephrology, neurodegenerative disorders and viral infections, all of which are connected to the loss of cell proliferation control, particularly CDK1, CDK2, CDK4 and CDK5. more particularly ATP:Proteinphosphotransferase p 34^{cdc2} (CDK1), in mammals, preferably in man.
Preferably, the indigoid bisindole derivatives are selected from indigo derivatives, isoindigo derivatives or indirubine derivatives.

In a preferred embodiment of the present invention, the indirubine derivate is a compound having the general formula (I) 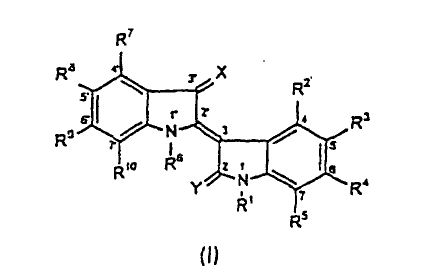 wherein the groups R¹ and R⁶ represent a hydrogen atom; R², R³, R⁴, R⁵, R⁷, R⁸, R⁹ and R¹⁰ can be the same or different and represent a hydrogen atom; a halogen atom; a hydroxy group; a nitroso group; a nitro group; an alkoxy group; a straight-chain or branched-chain alkyl group having 1 to 18 carbon atoms which can additionally carry one or more hydroxy and/or amino groups; a substituted or unsubstituted aryl group which can comprise one or more heteroatoms; a substituted or unsubstituted aralkyl group which can comprise one or more heteroatoms; a substituted or unsubstituted aryloxy-group which can comprise one or more heteroatoms; a substituted or unsubstituted methylenearyloxy group which can comprise one or more heteroatoms; a cycloalkyl group having 3 to 7 carbon atoms which can comprise one or more heteroatoms; a methylenecycloalkyl group having 3 to 7 carbon atoms which can comprise one or more heteroatoms; a trifluoromethyl group; a -COM group; a -COOM group; a -CH₂COOM group, wherein M is hydrogen, a straight-chain or branched-chain alkyl group having 1 to 18 carbon atoms which can additionally carry one or more hydroxy and/or amino groups, or an aryl group which can comprise one or more heteroatoms and can be substituted with one or more halogen atoms, one or more alkyl groups or one or more alkoxy groups; a -NR¹¹R¹² group, wherein R¹¹ and R¹² can be the same or different and represent a hydrogen atom, a straight-chain or branched-chain alkyl group having 1 to 18 carbon atoms which can additionally carry one or more hydroxy and/or amino groups, a substituted or unsubstituted aryl group which can comprise one or more heteroatoms, or an acyl group, or wherein the nitrogen atom is part of a cycloalkyl group having 3 to 7 carbon atoms which can comprise one or more heteroatom(s); a -CONR¹¹R¹² group, wherein R¹¹ and R¹² have the above definitions; a hydroxylamino group; a phosphate group; a phosphonate group; a sulfate group; a sulfonate group; a sulfonamide group ; a -SO₂NR¹¹R¹² group, wherein R¹' and R¹² have the above definitions; an azo group -N=N-R¹³, in which R¹³ represents an aromatic system which can be substituted by one or more carboxyl groups, phosphoryl groups or sulfonate groups; or a O-glycoside or a N-glycoside, wherein the glycoside is selected from monosaccharides or disaccharides; or R¹ and R⁵, and R⁶ and R¹⁰. respectively, form independently from each other a ring together having 1 to 4, optionally substituted, CH₂ groups; and X and Y can be the same or different and represent an oxygen atom; a sulfur atom; a selenium atom; a tellurium atom; a NR₁₄ group in which the group R¹⁴ represents a hydrogen atom, a straight-chain or branched-chain alkyl group having 1 to 18 carbon atoms which can be substituted by one or more carboxyl groups, phosphoryl groups or sulfonate groups, a substituted or unsubstituted aryl group which can comprise one or more heteroatoms, an aralkyl group, or a sulfonate group; or a NOR¹⁴ group, wherein the group R¹⁴ has the above definitions.

With respect to the benzene nuclei constituting the indirubine derivates of the the above general formula (I) one or more ring atoms can be replaced by nitrogen atoms. Further, one or more aromatic or non-aromatic ring systems which can comprise one or more heteroatoms independently of each other, can be condensed to the indirubine system. Furthermore, the indirubine derivatives having the above general formula (I) can also be bound to a polyethyleneglycolester or a polyethyleneglycolether by ester bondings or ether bondings, respectively.

In another embodiment of the present invention, the isoindigo derivate is a compound having the general formula (II) 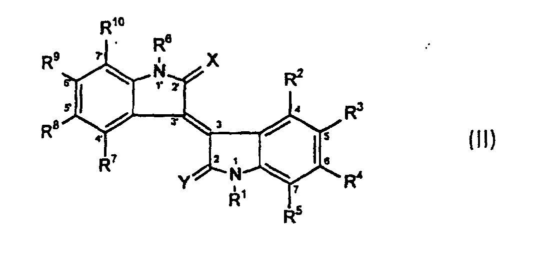 wherein R¹ to R¹⁴ and X and Y have the above definitions.

With respect to the benzene nuclei constituting the isoindigo derivates of the above general formula (II) one or more ring atoms can be replaced by nitrogen atoms. Further, one or more aromatic or non-aromatic ring systems which can comprise one or more heteroatoms independently of each other, can be condensed to the isoindigo system. Furthermore, the isoindigo derivatives having the above general formula (II) can be bound to a polyethyleneglycolester or a polyethyleneglycolether by ester bondings or ether bondings, respectively.

In a further embodiment of the present invention, the indigo derivate is a compound having the general formula (III) 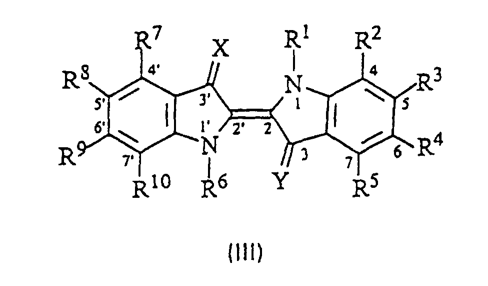 wherein R¹ to R¹⁴ and X and Y have the above definitions.

With respect to the benzene nuclei constituting the indigo derivates of the above general formula (III) one or more ring atoms can be replaced by nitrogen atoms. Further, one or more aromatic or non-aromatic ring systems which can comprise one or more heteroatoms independently of each other, can be condensed to the indigo system. Furthermore, the indigo derivatives having the above general formula (III) can be bound to a polyethyleneglycolester or a polyethyleneglycolether by ester bondings or ether bondings, respectively.

The above indigoid bisindole derivatives having the general formulas (I), (II) or (III) can also be in the form of their physiologically acceptable salts.

In the search for new selective inhibitors of cellular signalling pathways, it has surprisingly been found that indigoid bisindole derivatives are highly selective inhibitors of the enzyme complex p34^{cdc2}/cyclinB. An inhibition of p34^{cdc2}-kinase by indigoid bisindole derivatives is not described in the prior art. Surprisingly, it has turned out that indigoid bisindole derivatives are both highly selective and highly effective inhibitors of cdc2-kinase and other cyclin dependent kinases (CDK's), showing (C₅₀-values with the isolated enzyme down to submicromolar range. The inhibition of CDK-activities also is observed in cell culture, using human tumor cell lines. As an example, indirubin-3'-monoxime was found to inhibit histone H1 phosphorylation as a measure for CDK1/cyclin B activity after 24 h incubation of MCF-7 mammary carcinoma cells. Moreover, the content of cyclin B complex was significantly reduced. Cells arrested by serum deprivation after treatment for 24 h with indirubin-3'-monoxime in serum containing medium exhibited an arrest in G1-phase of the cell cycle at low micromolar concentrations of the substance. In concentrations ≥ 5 µM, an additional arrest at G2/M-phase of the cell cycle became apparent. Cells arrested in G2/m by Nocodazole treatment after release of the block exhibited a significant increase of G2/M arrested cells under treatment with ≥ 5 µM indirubin-3'-monoxime, resulting in a massive accumulation of cells in G2/M. Concomitantly with the observed intracellular effects, growth inhibition was induced in the same concentration range, resulting in an IC₅₀-value of 3.3 ±0.7 µM after 3 days incubation (IC₅₀ = concentration that induces 50% growth inhibition as compared to vehicle control). In addition, induction of apoptotic cell death was observed.

Based on the above mentioned selective inhibitory potency down to the nanomolar range, indirubine derivatives can be used for analytical biochemistry, especially for the study of cell cycle effects. Furthermore, these compounds can be used for the treatment of diseases in patients, which are connected to the loss of proliferation control without any restriction to these potential areas of application. These include psoriasis, cardiovascular diseases (stenosis, restenosis) (Brooks et al., J. Biol. Chem., 272, (1997), 29207-2921-1), infectious diseases (unicellular parasites (Trypanosoma, Toxoplasma, Plasmodium, etc.), fungi, etc.), nephrology (glomerulonephritis: Pippin et al., J. Clin. Invest., 100, (1997), 2512-2520), neurodegenerative disorders such as Alzheimer disease (Imahori, K., Uchida T., J. Biochem., 121, (1997), 179-188), viral infections such as cytomegalovirus (Bresnahan W. A. et al., Virology 231,(1997), 239-247) and HIV (Mancebo H.S.Y. et al., Genes & Dev., 11, (1997), 2633-2644).

The present invention is explained further by the following examples:

### Example 1: Synthesis of indirubine

To a solution of 0.42 g (2.4 mmol) of indoxyl acetate in 20 ml methanol under argon 0.35 g (2.4 mmol) of isatin and 0.55 g (5.2 mmol) of sodium carbonate are added. The mixture is stirred for 30 min at ambient temperature. After 24 h standing at ambient temperature, the reaction mixture is filtered off. The precipitate is washed with little methanol and water until the filtrate shows a neutral pH. Residual water is removed by storage in an evacuated exsiccator over potassium hydroxide. Recrystallisation from ethanol or pyridine gives deep purple crystals (Russell G.A., Kaupp G. (1969), J. Am. Chem. Soc., 91, 3851-9, modified).
Yield: 0.51 g (81%), fine, deep-purple needles, Fp: 341-343°C
CHN-analysis: (C₁₆H₁₀N₂O₂); MW: 262,26 g/mol; calc.: 73.3% C, 3.8% H, 10.7% N; found: 73.2% C, 4.0% H, 10.6% N
mass spectrum: m/z = 262: (M⁺, 100%), 234: (43%), 205 (25%), 158 (3%), 131 (4%), 103 (7%), 76 (3%)
¹H-NMR and ¹³C-NMR-spectrum are in accordance with the proposed structure. IR-spectrum: 3340 cm⁻¹: *v* (N-H), 1710 cm⁻¹: *v* (3'-C=O), 1650 cm⁻¹: *v* (2- C = O), 1590 cm⁻¹: *v* (C = C, aryl), 1450cm⁻¹: *v* (C = C, aryl), 745 cm⁻¹: *v* (aryl with four neighbouring H-atoms).
UV/Vis-spectrum (DMSO): 290 nm, 363 nm, 383 nm (shoulder), 551 nm

Essentially the same synthetic procedere was applied for the following Examples 2 to 9 and 11 and 12:

### Example 2: 5-lodoindirubine

Yield: 80%, fine, deep-purple needles, Fp: 334-335 °C (decomposition);
CHN-analysis (C₁₆H₉IN₂O₂); MG = 388.16 g/mol; calc.: 49.5% C, 2.3% H, 7.2% N; found.: 49.7% C, 2.5% H, 7.1% N;
Mass spectrum: 388 (M⁺, 100%), 360 (3%), 269 (9%), 261 (6%), 233 (16%), 205 (16%), 128 (1%);
¹H-NMR- and ¹³C-NMR-spectrum are in accordance with the proposed structure. UV/Vis-spectrum (DMSO): 370 nm, 386 nm (shoulder), 555 nm.

### Example 3: 5-Bromoindirubine

Yield: 70%, fine, deep-purple needles;
CHN-analysis (C₁₆H₉BrN₂O₂); MG = 341.16 g/mol, calc.: 56.3% C, 2.7% H, 8.2% N; found 56.4% C, 2.7% H, 8.2% N;
Mass spectrum: 342(M⁺, 100%), 340 (M⁺, 99%), 314 (18%), 262 (64%), 233 (34%), 205 (81%), 177 (10%);
¹H-NMR- and ¹³C-NMR-spectrum are in accordance with the proposed structure.

### Example 4: 5-Chloroindirubine

Yield: 95%, fine, deep-purple needles;
CHN-analysis (C₁₆H₉ClN₂O₂); MG = 296.70 g/mol; calc.: 49.5% C, 2.3% H, 7.2% N; found: 49.7% C, 2.5% H, 7.1% N;
Mass spectrum: m/z = 296 (M⁺, 100%), 268 (39%), 239 (8%), 233 (35%), 205 (50%), 177 (7%), 153 (6%), 137 (7%), 77 (7%), 120 (4%), 102 (6%), 77 (7%).
¹H-NMR- and ¹³C-NMR-spectrum are in accordance with the proposed structure.

### Example 5: 5-Fluoroindirubine

Yield: 92%, fine, deep-purple needles;
CHN-analysis (C₁₆H₉FN₂O₂), MG = 280.25 g/mol, calc.: 68.6% C, 3.2% H, 9.9% N; found: 68.0%C, 3.2% H, 9.9% N;
Mass spectrum: m/z = 281 (M⁺ + H⁺, 19%), 280 (M⁺. 100%), 252 (73%), 223 (32%), 176 (6%), 140 (7%), 121 (13%), 94 (4%), 76 (12%), 77 (7%), 57 (4%), 44(15%).
¹H-NMR- and ¹³C-NMR-spectrum are in accordance with the proposed structure.

### Example 6: 5-Methylindirubine:

Yield: 92%, fine, deep-purple needles;
CHN-analysis (C₁₇H₁₂N₂O₂), MG = 276.28 g/mol, calc.: 73.9% C, 4.4% H, 10.1% N; found: 73.8%C, 4.3% H, 10.2% N;
Mass spectrum: m/z = 276 (M⁺, 100%), 261 (10%), 248 (47%), 247 (53%), 220 (6%), 219 (18%), 205 (7%), 171 (4%), 165 (10%), 138 (4%), 133 (15%), 104 (7%), 77 (7%);
¹H-NMR- and ¹³C-NMR-spectrum are in accordance with the proposed structure.

### Example 7: 5-Nitroindirubine

Yield: 88%, fine, deep-purple needles;
CHN-analysis (C₁₆H₉N₃O₄), MG = 307.26 g/mol; calc.: 62.5% C, 3.0% H, 13.7% N; found: 62.4%C, 3.0% H, 13.3% N;
Mass spectrum: m/z = 307 (M⁺, 5%), 276 (10%), 262 (100%), 234 (23%), 205 (22%), 158 (6%), 131 (10), 104 (19%), 76 (12%), 50 (6%).
¹H-NMR- and ¹³C-NMR-spectrum are in accordance with the proposed structure.

### Example 8: 5'-Bromoindirubine

Yield: 92%, fine, deep-purple needles;
CHN-analysis (C₁₆H₉BrN₂O₂), MG = 341.16 g/mol, calc.: 56.3% C, 2.7% H, 8.2% N; found: 55.7%C, 2.5% H, 8.0% N.
¹H-NMR- and ¹³C-NMR-spectrum are in accordance with the proposed structure.

### Example 9: 5,5'-Dibromoindirubine

Yield: 94%, fine, deep-purple needles;
CHN-analysis (C₁₆H₈Br₂N₂O₂), MG = 420.06 g/mol; calc.: 45.7% C, 1.9% H, 6.7% N; found: 45.8% C, 2.0% H, 6.4% N.
¹H-NMR-spectrum is in accordance with the proposed structure.

### Example 10: Indirubine-3'-oxime

Indirubine-3'-oxime was synthesized by reaction of indirubine with hydroxylamine hydrochloride in a pyridine solution (Farbwerke vorm. Meister Lucius & Bruning in Hoechst a.M., Patentschrift des Reichspatentamtes Nr. 283726 (1913)). ¹³C-NMR-spectroscopy revealed the location of the hydroxyimino residue in 3'-Position (δ(C2) = 171.05 ppm; δ(C3') = 145.42 ppm; DMSO-d₆, RT)
Yield: 90 %, red crystals;
CHN-analysis (C₁₆H₁₁N₃O₂), MG = 277.30g/mol; calc.: 69.3% C, 4.0% H, 15.2 % N; found: 69.0% C, 4.0% H, 14.9% N;
¹H-NMR- and ¹³C-NMR-spectrum are in accordance with the proposed structure.

### Example 11: Indirubine-5-sulfonic acid

Yield: 76%, crystalline, deep-purple substance;
Mass spectrum: 388 (M⁺, 100%), 360 (3%), 269 (9%), 261 (6%), 233 (16%), 205 (16%), 128 (1%).
¹H-NMR- and ¹³C-NMR-spectrum are in accordance with the proposed structure.

Table 2 shows the structures of the compunds of Examples 1 to 11.

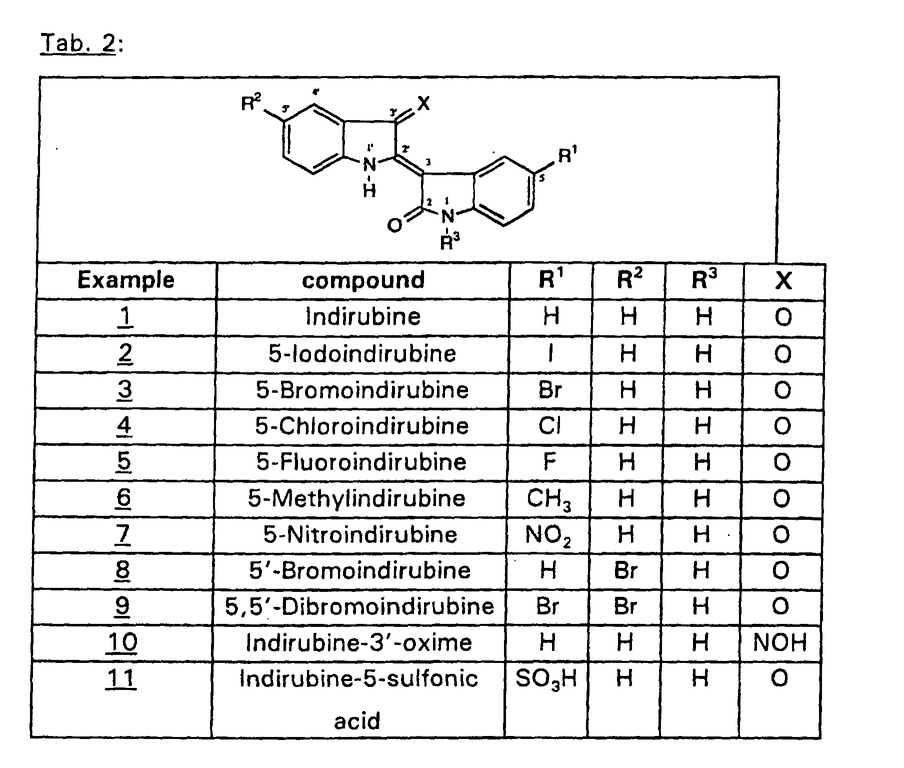

Table 3 shows the specificity of the compounds of Examples 1 to 11 (IC₅₀₋ values given in µM) to inhibit cdc2 kinase in comparison to other cellular kinases.

**Tab. 3:**

| **Example** | **cdc2** | **cdc25** | **PKA** | **PKC** |
|---|---|---|---|---|
| 1 | 2 | 150 | no Effect | no Effect |
| 2 | 0.3 | 130 | no Effect | no Effect |
| 3 | 0.35 | 32 | ND | ND |
| 4 | 0.40 | 55 | ND | ND |
| 5 | 2.0 | 70 | ND | ND |
| 6 | 0.8 | 60 | ND | ND |
| 7 | 2.2 | 60 | ND | ND |
| 8 | 6.5 | ND | ND | ND |
| 9 | 7 | ND | ND | ND |
| 10 | 0.18 | ND | ND | ND |
| 11 | 0.055 | 110 | ND | ND |
| ND: not dedected; PKA: cAMP-dependent protein kinase; | | | | |
| PKC: Ca²⁺- dependent protein kinase | | | | |

### Example 12: Isoindigo

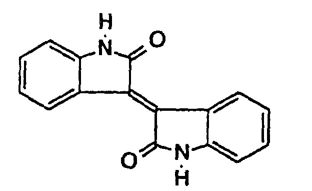

Isoindigo was synthesized by reaction of oxindole with isatin in acetic acid with addition af hydrochloric acid (Wahl A., Bayard P., Comptes Rendues Hebdomadaires des Seances de L'Academie des Sciences, 148, (1909), 716-719).
Yield: 84%, crystalline, brown substance;
CHN-analysis (C₁₆H₁₀N₂O₂), MG = 262.26 g/mol; calc.: 73.3% C, 3.8% H, 10,7% N; found: 73.0% C, 3.8% H, 10.9% N;
Mass spectrum: m/z = 262 (M⁺, 100%), 234 (85%), 220 (5%), 205 (18%), 190 (4%), 177 (5%), 151 (5%), 132 (17%), 103 (6%), 76 (4%), 32 (26%).
¹H-NMR- and ¹³C-NMR-spectrum are in accordance with the proposed structure.

Isoindigo shows an IC₅₀-value of 80µM for the p34^{cdc2}/cyclinB complex.

Table 4 shows the kinase inhibition selectivity of indigo, indirubin, 5-chloroindirubine, indirubine-3'-monoxim and indirubine-5 sulfonic acid. The indicated IC₅₀ values were calculated from respective dose response curves and are presented in µM.

**Tab. 4:**

| Enzyme | indigo | indirubine | 5-chloro-indirubine | indirubine-3'-monoxime | indirubine-5-sulfonic acid |
|---|---|---|---|---|---|
| CDK1/ cyclin B | > 1000.000 | 10.000 | 0.400 | 0.180 | 0.055 |
| CDK2/ cyclin A | 70.000 | 2.200 | 0.750 | 0.440 | 0.035 |
| CDK2/ cyclin E | > 1000.000 | 7.500 | 0.550 | 0.250 | 0.150 |
| CDK4/ cyclin D1 | > 100.000 | 12.000 | 6.500 | n.t. | 0.300 |
| CDK5/ p35 | > 100.000 | 5.500 | 0.800 | 0.100 | 0.065 |

## Claims

1. Use of indigoid bisindole derivatives for the manufacture of a medicament for inhibiting cyclin dependent kinases for the treatment of psoriasis, cardiovascular diseases, infectious diseases, nephrology, neurodegenerative disorders and viral infections, all of which are connected to the loss of cell proliferation control.

2. Use according to claim 1, wherein the cyclin dependent kinases are selected from CDK 1, CDK 2, CDK 4 or CDK5.

3. Use according to claim 1 or 2, wherein the indogoid bisindole derivatives are selected from indigo derivatives, isoindigo derivatives or indirubine derivatives.

4. Use according to claim 3, wherein the indirubine derivate is a compound having the general formula (I) 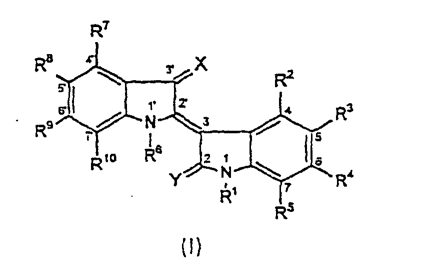 wherein the groups R¹ and R⁵ represent a hydrogen atom; R², R³, R⁴, R⁵, R⁷, R⁸, R⁹ and R¹⁰ can be the same or different and represent a hydrogen atom; a halogen atom; a hydroxy group; a nitroso group; a nitro group; an alkoxy group; a straight-chain or branched-chain alkyl group having 1 to 18 carbon atoms which can additionally carry one or more hydroxy and/or amino groups; a substituted or unsubstituted aryl group which can comprise one or more heteroatoms; a substituted or unsubstituted aralkyl group which can comprise one or more heteroatoms; a substituted or unsubstituted aryloxy group which can comprise one or more heteroatoms; a substituted or unsubstituted methylenearyloxy group which can comprise one or more heteroatoms; a cycloalkyl group having 3 to 7 carbon atoms which can comprise one or more heteroatoms; a methylenecycloalkyl group having 3 to 7 carbon atoms which can comprise one or more heteroatoms; a trifluoromethyl group; a-COM group; a -COOM group; a -CH₂COOM group, wherein M is hydrogen, a straight-chain or branched-chain alkyl group having 1 to 18 carbon atoms which can additionally carry one or more hydroxy and/or amino groups, or an aryl group which can comprise one or more heteroatoms and can be substituted with one or more halogen atoms, one or more alkyl groups or one or more alkoxy groups; a -NR¹¹R¹² group, wherein R¹¹ and R¹² can be the same or different and represent a hydrogen atom, a straight-chain or branched-chain alkyl group having 1 to 18 carbon atoms which can additionally carry one or more hydroxy and/or amino groups, a substituted or unsubstituted aryl group which can comprise one or more heteroatoms, or an acyl group, or wherein the nitrogen atom is part of a cycloalkyl group having 3 to 7 carbon atoms which can comprise one or more heteroatom(s); a -CONR¹¹R¹² group, wherein R¹¹ and R¹² have the above definitions; a hydroxylamino group; a phosphate group; a phosphonate group; a sulfate group; a sulfonate group; a sulfonamide group; a -SO₂NR¹¹R¹² group, wherein R¹¹ and R¹² have the above definitions; an azo group -N =N-R¹³, in which R¹³ represents an aromatic system which can be substituted by one or more carboxyl groups, phosphoryl groups or sulfonate groups; or a O-glycoside or a N-glycoside, wherein the glycoside is selected from monosaccharides or disaccharides; or R¹ and R⁵, and R⁶ and R¹⁰ respectively, form independently from each other a ring together having 1 to 4, optionally substituted, CH₂ groups; and X and Y can be the same or different and represent an oxygen atom; a sulfur atom; a selenium atom; a tellurium atom; a NR¹⁴ group in which the group R¹⁴ represents a hydrogen atom, a straight-chain or branched-chain alkyl group having 1 to 18 carbon atoms which can be substituted by one or more carboxyl groups, phosphoryl groups or sulfonate groups, a substituted or unsubstituted aryl group which can comprise one or more heteroatoms, an aralkyl group, or a sulfonate group; or a NOR¹⁴ group, wherein the group R¹⁴ has the above definitions.

5. Use according to claim 4, wherein one or more ring atoms of the benzene nuclei of the compound having the general formula (I) are replaced by nitrogen atoms.

6. Use according to claim 4 or 5, wherein one or more aromatic or non-aromatic ring systems which can comprise one or more heteroatoms independently of each other, are condensed to the indirubine system.

7. Use according to anyone of claims 4 to 6, wherein the compound having the general formula (I) is bound to a polyethyleneglycolester or a polyethyleneglycolether.

8. Use according to claim 3, wherein the isoindigo derivate is a compound having the general formula (II) 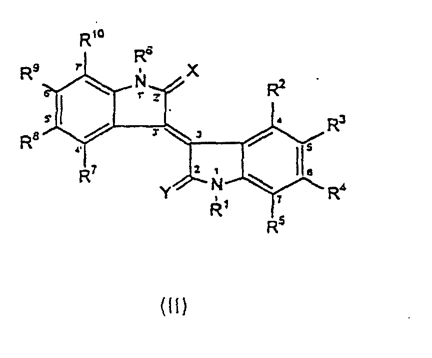 wherein R¹ to R¹⁴ and X and Y have the meanings as defined in claim 4.

9. Use according to claim 8, wherein one or more ring atoms of the benzene nuclei of the compound having the general formula (II) are replaced by nitrogen atoms.

10. Use according to claim 8 or 9, wherein one or more aromatic or non-aromatic ring systems which can comprise one or more heteroatoms independently of each other, are condensed to the isoindigo system.

11. Use according to anyone of claims 8 to 10, wherein the compound having the general formula (II) is bound to a polyethyleneglycolester or a polyethyleneglycolether.

12. Use according to claim 3, wherein the indigo derivate is a compound having the general formula (III) 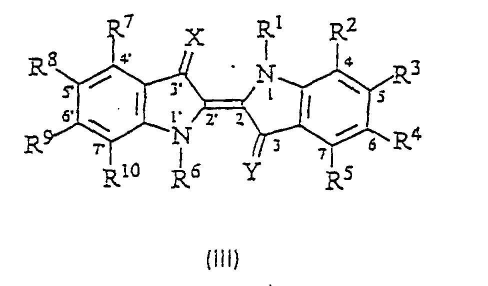 wherein R¹ to R¹⁴ and X and Y have the meanings as defined in claim 4.

13. Use according to claim 12, wherein one or more ring atoms of the benzene nuclei of the compound having the general formula (ill) are replaced by nitrogen atoms.

14. Use according to claim 12 or 13, wherein one or more aromatic or non-aromatic ring systems which can comprise one or more heteroatoms independently of each other, are condensed to the indigo system.

15. Use according to anyone of claims 12 to 14, wherein the compound having the general formula (III) is bound to a polyethyleneglycolester or a polyethyleneglycolether.

16. Use according to anyone of claims 1 to 15, wherein the indigoid bisindole derivative is in the form of a physiologically acceptable salt.

17. Use according to any one of claims 1 to 16, wherein the indigoid bisindole derivative is 5-fluoroindirubine.

18. Use according to any one of claims 1 to 16, wherein the indigoid bisindole derivative is 5-nitroindirubine.

19. Use according to any one of claims 1 to 16, wherein the indigoid bisindole derivative is 5,5'-dibromoindirubine.

## Patentansprüche

1. Verwendung von indigoiden Bisindol-Derivaten zur Herstellung eines Arzneimittels zur Hemmung cyclinabhängiger Kinasen bei der Behandlung von Psoriasis, Herzkreislauferkrankungen, infektiösen Erkrankungen, Nephrologie, neurodegenerativen Erkrankungen und viralen Infektionen, welche alle mit dem Verlust der Zellproliferationskontrolle verbunden sind.

2. Verwendung nach Anspruch 1, wobei die cyclinabhängigen Kinasen aus CDK 1, CDK 2, CDK 4 oder CDK 5 ausgewählt sind.

3. Verwendung nach Anspruch 1 oder 2, wobei die indigoiden Bisindol-Derivate aus Indigoderivaten, Isoindigoderivaten oder Indirubinderivaten ausgewählt sind.

4. Verwendung nach Anspruch 3, wobei das Indirubinderivat eine Verbindung mit der allgemeinen Formel (I) ist 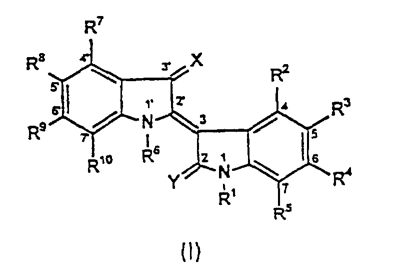 worin die Gruppen R¹ und R⁶ ein Wasserstoffatom darstellen, R², R³, R⁴, R⁵, R⁷, R⁸, R⁹ und R¹⁰ gleich oder unterschiedlich sein können und ein Wasserstoffatom, ein Halogenatom, eine Hydroxygruppe, eine Nitrosogruppe, eine Nitrogruppe, eine Alkoxygruppe, eine geradkettige oder verzweigtkettige Alkylgruppe mit 1 bis 18 Kohlenstoffatomen, welche zusätzlich eine oder mehrere Hydroxy- und/oder Aminogruppen tragen kann, eine substituierte oder unsubstituierte Arylgruppe, welche ein oder mehrere Heteroatome umfassen kann, eine substituierte oder unsubstituierte Aralkylgruppe, welche ein oder mehrere Heteroatome umfassen kann, eine substituierte oder unsubstituierte Aryloxygruppe, welche ein oder mehrere Heteroatome umfassen kann, eine substituierte oder unsubstituierte Methylenaryloxygruppe, welche ein oder mehrere Heteroatome umfassen kann, eine Cycloalkylgruppe mit 3 bis 7 Kohlenstoffatomen, welche ein oder mehrere Heteroatome umfassen kann, eine Methylencycloalkylgruppe mit 3 bis 7 Kohlenstoffatomen, welche ein oder mehrere Heteroatome umfassen kann, eine Trifluormethylgruppe, eine -COM-Gruppe, eine -COOM-Gruppe, eine -CH₂COOM-Gruppe, worin M ein Wasserstoffatom, eine geradkettige oder verzweigtkettige Alkylgruppe mit 1 bis 18 Kohlenstoffatomen, welche zusätzlich eine oder mehrere Hydroxy- und/oder Aminogruppen tragen kann, oder eine Arylgruppe ist, welche ein oder mehrere Heteroatome umfassen kann und mit einem oder mehreren Halogenatomen, einer oder mehreren Alkylgruppen oder einer oder mehreren Alkoxygruppen substituiert sein kann, eine -NR¹¹R¹²-Gruppe, worin R¹¹ und R¹² gleich oder unterschiedlich sein können und ein Wasserstoffatom, eine geradkettige oder verzweigtkettige Alkylgruppe mit 1 bis 18 Kohlenstoffatomen, welche zusätzlich eine oder mehrere Hydroxy-und/oder Aminogruppen tragen kann, eine substituierte oder unsubstituierte Arylgruppe, welche ein oder mehrere Heteroatome umfassen kann, oder eine Acylgruppe darstellen, oder worin das Stickstoffatom Teil einer Cycloalkylgruppe mit 3 bis 7 Kohlenstoffatomen ist, welche ein oder mehrere Heteroatome umfassen kann, eine -CONR¹¹R¹²-Gruppe, worin R¹¹ und R¹² die vorstehenden Definitionen haben, eine Hydroxylaminogruppe, eine Phosphatgruppe, eine Phosphonatgruppe, eine Sulfatgruppe, eine Sulfonatgruppe, eine Sulfonamidgruppe, eine -SO₂NR¹¹R¹²-Gwppe, worin R¹¹ und R¹² die vorstehenden Definitionen haben, eine Azogruppe -N=N-R¹³, in welcher R¹³ ein aromatisches System darstellt, welches mit einer oder mehreren Carboxylgruppen, Phosphorylgruppen oder Sulfonatgruppen substituiert sein kann, oder ein O-Glycosid oder ein N-Glycosid, wobei das Glycosid aus Monosacchariden oder Disacchariden ausgewählt ist, darstellen, oder R¹ und R⁵ bzw. R⁶ und R¹⁰ unabhängig voneinander miteinander einen Ring mit 1 bis 4, gegebenenfalls substituierten CH₂-Gruppen bilden, und X und Y gleich oder unterschiedlich sein können und ein Sauerstoffatom, ein Schwefelatom, ein Selenatom, ein Telluratom, eine NR¹⁴-Gruppe, in welcher die Gruppe R¹⁴ ein Wasserstoffatom, eine geradkettige oder verzweigtkettige Alkylgruppe mit 1 bis 18 Kohlenstoffatomen, welche mit einer oder mehreren Carboxylgruppen, Phosphorylgruppen oder Sulfonatgruppen substituiert sein kann, eine substituierte oder unsubstituierte Arylgruppe, welche ein oder mehrere Heteroatome umfassen kann, eine Aralkylgruppe oder eine Sulfonatgruppe darstellt, oder eine NOR¹⁴-Gruppe, worin die Gruppe R¹⁴ die vorstehenden Definitionen hat, darstellen.

5. Verwendung nach Anspruch 4, wobei ein oder mehrere Ringatome der Benzolkerne der Verbindung mit der allgemeinen Formel (I) durch Stickstoffatome ersetzt sind.

6. Verwendung nach Anspruch 4 oder 5, wobei ein oder mehrere aromatische oder nicht-aromatische Ringsysteme, welche ein oder mehrere Heteroatome unabhängig voneinander umfassen können, an das Indirubinsystem kondensiert sind.

7. Verwendung nach einem der Ansprüche 4 bis 6, wobei die Verbindung mit der allgemeinen Formel (I) an einen Polyethylenglykolester oder einen Polyethylenglykolether gebunden ist.

8. Verwendung nach Anspruch 3, wobei das Isoindigoderivat eine Verbindung mit der allgemeinen Formel (II) ist 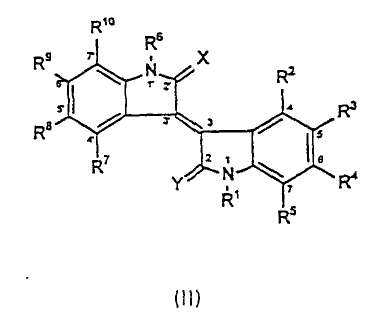 worin R¹ bis R¹⁴ und X und Y die in Anspruch 4 definierten Bedeutungen haben.

9. Verwendung nach Anspruch 8, wobei ein oder mehrere Ringatome der Benzolkerne der Verbindung mit der allgemeinen Formel (II) durch Stickstoffatome ersetzt sind.

10. Verwendung nach Anspruch 8 oder 9, wobei ein oder mehrere aromatische oder nicht-aromatische Ringsysteme, welche ein oder mehrere Heteroatome unabhängig voneinander umfassen können, an das Isoindigosystem kondensiert sind.

11. Verwendung nach einem der Ansprüche 8 bis 10, wobei die Verbindung mit der allgemeinen Formel (II) an einen Polyethylenglykolester oder einen Polyethylenglykolether gebunden ist.

12. Verwendung nach Anspruch 3, wobei das Indigoderivat eine Verbindung mit der allgemeinen Formel (III) ist 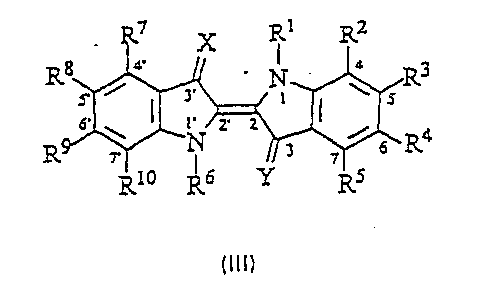 worin R¹ bis R¹⁴ und X und Y die in Anspruch 4 definierten Bedeutungen haben.

13. Verwendung nach Anspruch 12, wobei ein oder mehrere Ringatome der Benzolkerne der Verbindung mit der allgemeinen Formel (III) durch Stickstoffatome ersetzt sind.

14. Verwendung nach Anspruch 12 oder 13, wobei ein oder mehrere aromatische oder nicht-aromatische Ringsysteme, welche ein oder mehrere Heteroatome unabhängig voneinander umfassen können, an das Indigosystem kondensiert sind.

15. Verwendung nach einem der Ansprüche 12 bis 14, wobei die Verbindung mit der allgemeinen Formel (III) an einen Polyethylenglykolester oder einen Polyethylenglykolether gebunden ist.

16. Verwendung nach einem der Ansprüche 1 bis 15, wobei das indigoide Bisindol-Derivat in der Form eines physiologisch verträglichen Salzes vorliegt.

17. Verwendung nach einem der Ansprüche 1 bis 16, wobei das indigoide Bisindol-Derivat 5-Fluorindirubin ist.

18. Verwendung nach einem der Ansprüche 1 bis 16, wobei das indigoide Bisindol-Derivat 5-Nitroindirubin ist.

19. Verwendung nach einem der Ansprüche 1 bis 16, wobei das indigoide Bisindol-Derivat 5,5'-Dibromindirubin ist.

## Revendications

1. Utilisation de dérivés bisindoliques d'indigoïdes pour la production d'un médicament destiné à inhiber des kinases dépendant de cyclines, pour le traitement du psoriasis, de maladies cardiovasculaires, de maladies infectieuses, de maladies nèphrologiques, de troubles neurodégénératifs et d'infections virales, toutes ces affections étant en rapport avec la perte de régulation de la prolifération cellulaire.

2. Utilisation suivant la revendication 1, dans laquelle les kinases dépendant de cyclines sont choisies entre CDK 1, CDK 2, CDK 4 et CDK 5.

3. Utilisation suivant la revendication 1 ou 2, dans laquelle les dérivés bisindoliques d'indigoïdes sont choisis entre des dérivés d'indigo, des dérivés d'iso-indigo et des dérivés d'indirubine.

4. Utilisation suivant la revendication 3, dans laquelle le dérivé d'indirubine est un composé répendant à la formule générale (I) 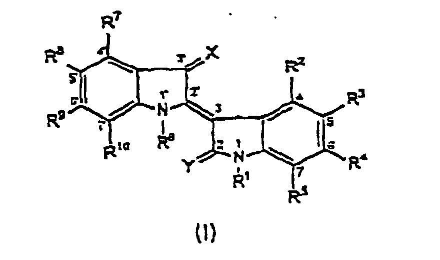 dans laquelle les groupes R¹ et R⁶ représentent un atome d'hydrogène ; R², R³, R⁴, R⁵, R⁷, R⁸, R⁹ et R¹⁰ peuvent être identiques ou différents et représentent un atome d'hydrogène ; un atome d'halogène ; un groupe hydroxy ; un groupe nitroso ; un groupe nitro ; un groupe alkoxy ; un groupe alkyle à chaîne droite ou à chaîne ramifiée ayant 1 à 18 atomes de carbone qui peut porter en outre un ou plusieurs groupes hydroxy et/ou amino ; un groupe aryle substitué ou non substitué qui peut comprendre un ou plusieurs hétéroatomes ; un groupe aralkyle substitué ou non substitué qui peut comprendre un ou plusieurs hétéroatomes ; un groupe aryloxy substitué ou non substitué qui peut comprendre un ou plusieurs hétéroatomes ; un groupe méthylène-aryloxy substitué ou non substitué qui peut comprendre un ou plusieurs hétéroatomes ; un groupe cycloalkyle ayant 3 à 7 atomes de carbone qui peut comprendre un ou plusieurs hétéroatomes ; un groupe méthylènecycloalkyle ayant 3 à 7 atomes de carbone qui peut comprendre un ou plusieurs hétéroatomes ; un groupe trifluorométhyle ; un groupe -COM ; un groupe -COOM ; un groupe -CH₂COOM, dans lequel M représente un atome d'hydrogène, un groupe alkyle à chaîne droite ou à chaîne ramifiée ayant 1 à 18 atomes de carbone qui peut porter en outre un ou plusieurs groupes hydroxy et/ou amino, ou un groupe aryle qui peut comprendre un ou plusieurs hétéroatomes et qui peut être substitué avec un ou plusieurs atomes d'halogènes, un ou plusieurs groupes alkyle ou un ou plusieurs groupes alkoxy ; un groupe -NR¹¹R¹², dans lequel R¹¹ et R¹² peuvent être identiques ou différents et représentent un atome d'hydrogène, un groupe alkyle à chaîne droite ou à chaîne ramifiée ayant 1 à 18 atomes de carbone qui peut porter en outre un ou plusieurs groupes hydroxy et/ou amino, un groupe aryle substitué ou non substitué qui peut comprendre un ou plusieurs hétéroatomes, ou un groupe acyle, ou dans lequel l'atome d'azote fait partie d'un groupe cycloalkyle ayant 3 à 7 atomes de carbone, qui peut comprendre un ou plusieurs hétéroatomes ; un groupe -CONR¹¹R¹², dans lequel R¹¹ et R¹² répondent aux définitions précitées ; un groupe hydroxylamino ; un groupe phosphate ; un groupe phosphonate ; un groupe sulfate ; un groupe sulfonate ; un groupe sulfonamide ; un groupe -SO₂NR¹¹R¹², dans lequel R¹¹ et R¹² répondent aux définitions précitées ; un groupe azo de formule -N=N-R¹³, dans laquelle R¹³ représente un système aromatique qui peut être substitué avec un ou plusieurs groupes carboxyle, groupes phosphoryle ou groupes sulfonate ; ou un O-glycoside ou N-glycoside, dans lequel le glycoside est choisi entre des monosaccharides et disaccharides ; ou bien R¹ et R⁵, et R⁶ et R¹⁰, respectivement, forment, indépendamment l'un de l'autre, un noyau comprenant au total 1 à 4 groupes CH₂ facultativement substitués ; et X et Y peuvent être identiques ou différents et représentent un atome d'oxygène ; un atome de soufre ; un atome de sélénium ; un atome de tellure ; un groupe NR¹⁴ dans lequel le groupe R¹⁴ représente un atome d'hydrogène, un groupe alkyle à chaîne droite ou à chaîne ramifiée ayant 1 à 18 atomes de carbone qui peut être substitué avec un ou plusieurs groupes carboxyle, groupes phosphoryle ou groupes sulfonate, un groupe aryle substitué ou non substitué qui peut comprendre un ou plusieurs hétéroatomes, un groupe aralkyle ou un groupe sulfonate ; ou un groupe NOR¹⁴, dans lequel le groupe R¹⁴ répond aux définitions précitées.

5. Utilisation suivant la revendication 4, dans laquelle un ou plusieurs atomes de noyau des noyaux benzéniques du composé répondant à la formule générale (I) sont remplacés par des atomes d'azote.

6. Utilisation suivant la revendication 4 ou 5, dans laquelle un ou plusieurs systèmes de noyaux aromatiques ou non aromatiques qui peuvent comprendre un ou plusieurs hétéroatomes, indépendamment les uns des autres, sont condensés au système indirubine;

7. Utilisation suivant l'une quelconque des revendications 4 à 6, dans laquelle le composé répondant à la formule générale (I) est lié à un ester de polyéthylène-glycol ou un éther de polyéthylène-glycol.

8. Utilisation suivant la revendication 3, dans laquelle le dérivé d'iso-indigo est un composé répondant à la formule générale (II) 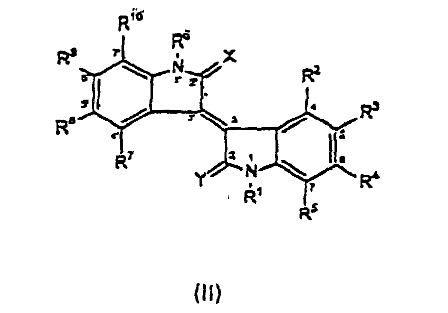 dans laquelle R¹ à R¹⁴ et X et Y répondent aux définitions figurant dans la revendication 4.

9. Utilisation suivant la revendication 8, dans laquelle un ou plusieurs atomes de noyau des noyaux benzéniques du composé répondant à la formule générale (II) sont remplacés par des atomes d'azote.

10. Utilisation suivant la revendication 8 ou 9, dans laquelle un ou plusieurs systèmes de noyaux aromatiques ou non aromatiques qui peuvent comprendre un ou plusieurs hétéroatomes, indépendamment les uns des autres, sont condensés au système iso-indigo.

11. Utilisation suivant l'une quelconque des revendications 8 à 10, dans laquelle le composé répondant à la formule générale (II) est lié à un ester de polyéthylène-glycol ou un éther de polyéthylène-glycol.

12. Utilisation suivant la revendication 3, dans laquelle le dérivé d'indigo est un composé répondant à la formule générale (III) 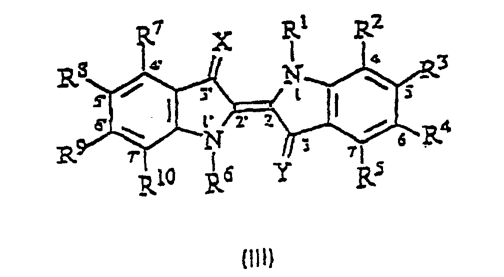 dans laquelle R¹ à R¹⁴ et X et Y répondent aux définitions figurant dans la revendication 4.

13. Utilisation suivant la revendication 12, dans laquelle un ou plusieurs atomes de noyau des noyaux benzéniques du composé répondant à la formule générale (III) sont remplacés par des atomes d'azote.

14. Utilisation suivant la revendication 12 ou 13, dans laquelle un ou plusieurs systèmes de noyaux aromatiques ou non aromatiques qui peuvent comprendre un ou plusieurs hétéroatomes, indépendamment les uns des autres, sont condensés au système indigo.

15. Utilisation suivant l'une quelconque des revendications 12 à 14, dans laquelle le composé répondant à la formule générale (III) est lié à un ester de polyéthylène-glycol ou un éther de polyéthylène-glycol.

16. Utilisation suivant l'une quelconque des revendications 1 à 15, dans laquelle le dérivé bisindolique d'indigoide est sous forme d'un sel physiologiquement acceptable.

17. Utilisation suivant l'une quelconque des revendications 1 à 16, dans laquelle le dérivé bisindolique d'indigoïde est la 5-fluoro-indirubine.

18. Utilisation suivant l'une quelconque des revendications 1 à 16, dans laquelle le dérivé bisindolique d'ingoïde est la 5-nitro-indirubine.

19. Utilisation suivant l'une quelconque des revendications 1 à 16, dans laquelle le dérivé bisindolique d'indigoïde est la 5,5'-dibromo-indirubine.
